# EUROPÄISCHE PATENTSCHRIFT

(11) **EP 0 674 734 B1**
(45) Veröffentlichungstag und Bekanntmachung des Hinweises auf die Patenterteilung: **28.05.1997**
(21) Anmeldenummer: 94901951.7
(22) Anmeldetag: 02.12.1993
(51) Int. Cl.: E01H 4/02

(54) **PISTENPFLEGEVORRICHTUNG**
SKI SLOPE MAINTENANCE DEVICE
DISPOSITIF POUR ENTRETENIR DES PISTES DE SKI

(30) Priorität: 21.12.1992 DE 9217472 U
(43) Veröffentlichungstag der Anmeldung: 04.10.1995
(73) Patentinhaber: Kässbohrer Geländefahrzeug GmbH, 89250 Senden (DE)
(72) Erfinder: HAUG, Walter, D-89134 Blaustein (DE)
(74) Vertreter: Grünecker, Kinkeldey, Stockmair & Schwanhäusser Anwaltssozietät
(86) Internationale Anmeldenummer: EP9303396
(87) Internationale Veröffentlichungsnummer: WO9415028

(56) Entgegenhaltungen:
- WO-A-81/03353
- DE-A- 2 946 796
- DE-A- 3 025 312
- DE-U- 9 212 233

## Beschreibung

Die Erfindung betrifft eine Pistenpflegevorrichtung zum Anbau an ein Fahrzeug mittels einer Kupplungseinrichtung mit einem aus wenigstens einem Querträger und zwei Längsträgern gebildeten Tragrahmen, an dem eine höhenverstellbare Schneefräse und ein Glättebrett gelagert sind, wobei der Tragrahmen an der Kupplungseinrichtung um eine im wesentlichen horizontale Längs- und eine im wesentlichen hcrizontale Querachse verschwenkbar gelagert ist.

Eine solche Pistenpflegevorrichtung ist aus der DE-A-29 46 796 bekannt. In dieser Druckschrift wird die Pistenpflegevorrichtung über eine Zugstange an einem Heck eines Fahrzeugs angekuppelt. Am hinteren Ende der Zugstange ist ein Tragrahmen um eine horizontale Quer- und eine horizontale Längsachse mittels zweier Längsträgerenden verschwenkbar gelagert. Der Tragrahmen ist in Seitenansicht im wesentlichen V-förmig ausgebildet, wobei an seinem hinteren Ende ein Glättebrett und benachbart zu diesem eine Schneefräse verschwenkbar gelagert sind. Mittels zweier Stelleinrichtungen zwischen Tragrahmen und Schneefräse bzw. Glättebrett ist die Schneefräse höhenverstellbar und ein Anstellwinkel des Glättebretts veränderlich. Dem Glättebrett ist ein Finisher nachgeordnet.

Nachteilig bei der aus der DE-A-29 46 796 bekannten Pistenpflegevorrichtung ist, daß deren Baulänge, die für ein Hochschwenken der Pistenpflegevorrichtung in eine Bereitschaftsstellung notwendig ist, relativ groß ist. Dadurch ist die Manövrierbarkeit des Fahrzeugs eingeschränkt, indem insbesondere nur Kurvenfahrten mit relativ großem Radius möglich sind. Auf den Pisten ist es daher schwierig, diese durch direkt nebeneinanderliegende Durchfahrten mit der Pistenpflegevorrichtung aufzubereiten.

Durch die Anordnung der Schneefräse als "geschobene" Schneefräse, wird weiterhin in nachteiliger Weise die Lenkung der Pistenpflegevorrichtung erschwert, da durch die geschobene Anordnung die Nachlaufeigenschaften der Pistenpflegevorrichtung ungünstig sind. Ein weiterer Nachteil ist die Anordnung des Tragrahmens als in Richtung zum Fanrzeug offenes U, wodurch die Steifigkeit des Tragrahmens ungünstig beeinflußt wird.

Der Erfindung liegt daher die Aufgabe zugrunde, eine Pistenpflegevorrichtung der eingangs genannten Art im Hinblick auf die Handhabbarkeit und die Kompaktheit bei gleichzeitiger Erhöhung der Steifigkeit zu verbessern.

Diese Aufgabe wird bei einer Pistenpflegevorrichtung mit den Merkmalen des Oberbegriffs des Anspruchs 1 dadurch gelöst, daß die Schneefräse geschleppt von vorderen Enden der Längsträger an diesen verschwenkbar gelagert ist und der Querträger zur Lagerung des Tragrahmens der Kupplungseinrichtung zugeordnet ist.

Durch die geschleppte Anordnung der Schneefräse an einer Heck- oder Frontseite eines Fahrzeugs wird die Handhabbarkeit der Pistenpflegevorrichtung insgesamt verbessert. Der Tragrahmen ist direkt ohne eine Zugstange über die Kupplungseinrichtung in eine Bereitschaftsstellung und zurück in eine Einsatzstellung verschwenkbar. Die Baulänge der Pistenpflegevorrichtung ist dadurch erheblich verringert. Aufgrunddessen ist ebenfalls der Kurvenradius, der mit der Pistenpflegevorrichtung durchfahren werden kann, verringert, so daß die aufbereiteten Abschnitte der Piste direkt nebeneinander oder, falls erwünscht, auch teilweise überlappend zueinander angeordnet sind, ohne daß ein Rangieren des Fahrzeugs mit angebauter Pistenpflegevorrichtung notwendig ist. Durch die Zuordnung des Querträgers zur Kupplungseinrichtung ist der Tragrahmen der Pistenpflegevorrichtung insbesondere am Angriffspunkt der vom Fahrzeug ausgeübten Kraft in günstiger Weise versteift. Die Krafteinleitung erfolgt in den Querträger so daß im Gegensatz zu der Krafteinleitung in die voneinander beabstandeten Längsträger, wie es in der DE-A-29 46 796 beschrieben ist, die Kupplungseinrichtung eine geringere Breite aufweist und einfacher ausgebildet ist. Durch die Anlenkung der Schneefräse an den vorderen Enden der Längsträger ist die Gewichtsverteilung im Tragrahmen dahingehend verbessert, daß die auf die im wesentlichen horizontale Querachse der Kupplungseinrichtung einwirkenden Lastarme relativ kurz sind, so daß ein Verschwenken der Pistenpflegevorrichtung in ihre Bereitschaftsstellung erleichtert ist.

Bei einer Weiterbildung der Erfindung verläuft der Querträger zwischen den Längsträgern und ist im wesentlichen mittig an der Kupplungseinrichtung verschwenkbar gelagert. Auf diese Weise ist der Tragrahmen in einfacher Weise versteift und der Tragrahmen ist mittels des Querträgers in einfacher Weise zwischen einer Bereitschaftsstellung und einer Einsatzstellung verschwenkbar gelagert.

Weiterhin ist günstig, wenn die Längsträger in Seitenansicht in etwa U-förmig mit jeweils einem U-Steg und einem vorderen und einem hinteren U-Schenkel ausgebildet sind.Dadurch sind Schneefräser und Glättebrett in einfacher Weise an den Enden der U-Schenkel anordbar, wobei das Glättebrett der Schneefräse nachgeordnet ist. Entsprechend zur Länge der U-Schenkel ergibt sich die Höhenverstellung der Schneefräse, die bis benachbart zum U-Steg anhebbar ist. Eine maximale Aushubhöhe der Schneefräse kann beispielsweise bei einer Ausführungsform der Erfindung 250 mm betragen.

Bei einer weiteren Ausführungsform der Erfindung schließt der der Schneefräse zugeordnete, vordere U-Schenkel im wesentlichen einen rechten Winkel und der dem Glättebrett zugeordnete, hintere U-Schenkel einen stumpfen Winkel mit dem U-Steg ein. Dadurch ist der Tragrahmen direkt benachbart zum vorderen U-Schenkel an der Kupplungseinrichtung lagerbar, so daß der Tragrahmen beim Verschwenken zwischen Bereitschaftund Einsatzstellung einen relativ kleinen Bereich überstreicht. Gleichzeitig ist das Glättebrett in einem ausreichenden Abstand zur Schneefräse angeordnet.

Um die Schneefräse in einfacher Weise relativ zum Tragrahmen verschwenkbar zu lagern, ist am freien Ende des vorderen U-Schenkels eine Lagerhülse zur Aufnahme eines um eine im wesentlichen horizontale Längsachse verschwenkbaren Lagerbolzen angeordnet, an dessen hinterem Ende die Schneefräse um eine im wesentlichen horizontale Querachse verschwenkbar gelagert ist. Auf diese Weise kann die Schneefräse Unebenheiten der Piste zumindest teilweise folgen.

Die Schneefräse umfaßt beispielsweise einen Fräsrahmen und wenigstens eine am Fräsrahmen drehbar gelagerte Fräswelle, wobei der Fräsrahmen an den vorderen U-Schenkeln angelenkt ist. Der Fräsrahmen dient in einfacher Weise sowohl zur Lagerung der fräswelle als auch zur Anlenkung am Tragrahmen.

Um von an der Fräswelle angeordneten Messern aufgeworfenen Schnee und/oder aufgeworfene Bruchstücke gefrorenen Schnees oder Eises zusätzlich zu zerkleinern und einen geringen Schneeauswurf nach vorne zu erzielen und einen Schneeüberwurf über die Schneefräse zu verhindern, weist der Fräsrahmen eine im wesentlichen halbkreisförmige Prallverkleidung, an deren seitlichen Enden die Fräswelle drehbar gelagert ist, und zwei zwischen aen U-Schenkelenden und der Prallverkleidung angeordnete Längslenker auf. Durch die Längslenker wird der Abstand von Fräswelle zum vorderen U-Schenkelende so bestimmt, daß die Schneefräse insgesamt optimal verschwenkbar ist.

Zur Vereinfachung der Höhenverstellung der Schneefräse ist zwischen Längslenker und U-Steg wenigstens eine Stelleinrichtung angeordnet. Durch die Stelleinrichtung ist sowohl die Frästiefe verstellbar als auch die Steigfähigkeit des Fahrzeugs bei angebauter Pistenpflegevorrichtung durch den von der Schneefräse ausgeübten Anpreßdruck erhöhbar. Die Schneefräse kann beispielsweise so weit in Richtung des Tragrahmens angehoben werden, bis die Prallverkleidung von unten am U-Steg anliegt.

Zur Erleichterung der Anordnung der Stelleinrichtung ist es von Vorteil, wenn der vordere U-Schenkel mit dem U-Steg über einen unter einem spitzen Winkel nach außen abgeknickten Verbindungsabschnitt verbunden ist. Auf diese Weise ist die Stelleinrichtung parallel zur Verstellrichtung der Schneefräse anordbar, wobei ein oberes Ende der Stelleinrichtung, beispielsweise seitlich am U-Steg verschwenkbar gelagert ist. Dadurch ist ein Zugriff auf die Stelleinrichtung leicht möglich, so daß sowohl entsprechende Verbindungen zur Betätigung der Stelleinrichtung als auch deren Wartung in einfacher Weise möglich sind.

Um die Handhabung der Pistenpflegevorrichtung weiter zu erleichtern, ist es insbesondere günstig, wenn benachbart zu den Verbindungsabschnitten der vorderen U-Schenkel zwischen den U-Stegen der Querträger angeordnet ist und an der Kupplungseinrichtung um die im wesentlichen horizontale Querachse verschwenkbar gelagert ist.

In diesem Zusammenhang ist es weiterhin von Vorteil, wenn der Querträger über zwei in etwa in Richtung des freien Endes der vorderen U-Schenkel verlaufende Lagerlaschen an der Kupplungseinrichtung gelagert ist. Die horizontale Querachse ist sowohl in Längsrichtung der Pistenpflegevorrichtung hinter der horizontalen Längsachse als auch bei einer anderen Ausführungsform unterhalb der horizontalen Längsachse anordenbar. Die Lagerlaschen sind beispielsweise an zwei Enden eines Lagerbolzens, der in der Kupplungseinrichtung drehbar gelagert ist, aufsteckbar.

Um den Tragrahmen bei einer einfachen Ausführungsform durch Verschwenken der Kupplungseinrichtung durch das Fahrzeug in Richtung zur Pistenoberfläche zu drücken, ist es günstig, wenn an der Kupplungseinrichtung ein Anschlag für den Querträger angeordnet ist.

Bei einer vorteilhaften Ausführungsform der Prallverkleidung der Fräswelle ist diese aus einem Hohlprofil mit in diesem im wesentlichen parallel zur Fräswelle verlaufenden Versteifungsrohren gebildet, wobei die Prallverkleidung seitlich durch im wesentlichen senkrecht zur Fräswelle verlaufende Schürzen abgedeckt ist. Diese dienen gleichzeitig zur Lagerung der Fräswelle. Aufgrund des Hohlprofils und der Versteifungsrohre ist die Prallverkleidung stabil ausgeführt und weist ein geringes Gewicht auf. Durch die seitlichen Schürzen wird eine Randwallbildung durch aufgehäuften Schnee od.dgl. verhindert. Die Prallverkleidung kann beispielsweise aus einem Kunststoffmaterial oder auch aus Metallblechen hergestellt sein. Außerdem kann die der Fräswelle zugeordnete Unterseite der Prallverkleidung aus einem anderen Material als die nach außen weisende Oberseite gebildet sein.

Um die Flexibilität der Schneefräse zu erhöhen, ist diese bei einer vorteilhaften Ausführungsform aus zwei Fräswellen mit entsprechenden Fräsrahmen gebildet, wobei die Längslenker eines jeden Fräsrahmens in Querrichtung im wesentlichen mittig an diesen angeordnet sind. Die beiden Fräswellen können insbesondere einheitlich ausgebildet sein, so daß die Herstellungskosten verringert sind. Durch die getrennte Lagerung der einzelnen Fräsrahmen und der entsprechenden Fräswellen über die zugeordneten Längslenker ist die Schneefräse an Pistenunebenheiten anpaßbar. Auf diese Weise sind insbesondere Randbereiche der Pisten leichter zu bearbeiten und durch die nicht vollständige Einebnung der Piste in Richtung der Fräsewellen ist beispielsweise für einen Skifahrer eine interessantere Pistenführung möglich, statt einer "langweiligen" ebenen Piste. Eine geteilte Fräswelle ist beispielsweise aus "Logan Manufacturing's Contour Grooming / Flex Tiller" (1990) bekannt. Allerdings wird bei dieser Pistenpflegevorrichtung die Fräswelle aus drei miteinander gelenkig verbundenen Fräswellen gebildet. Durch die beiden Gelenke ist der Wirkungsgrad der Schneefräse reduziert und eine separate Verstellung jeder Fräswelle ist nicht möglich.

Dabei ist es insbesondere von Vorteil, wenn jeder Fräswelle eine Antriebseinrichtung zugeordnet ist. Durch diese sind die Fräswellendrehzahlen unabhängig voneinander, beispielsweise im Bereich von 700 bis 1400 U/min regulierbar. Die Antriebseinrichtungen sind als Elektromotore, hydrostatische Antriebe od.dgl. ausbildbar und können direkt ein Ende einer Fräswelle antreiben.

Dabei ist es weiterhin von Vorteil, wenn die Antriebseinrichtungen an außenliegenden, seitlichen Enden der Fräswellen angeordnet sind. Auf diese Weise ist ein möglichst geringer Mittelabstand der Fräswellen, beispielsweise in der Größenordnung von 100 mm realisierbar. Zwischen den beiden Fräswellen ist aufgrund der separaten Lagerung jeder Fräswelle eine Gelenkverbindung und aufgrund der separaten Antriebseinrichtungen eine Antriebsverbindung nicht notwendig. Auf diese Weise wird die gesamte Pistenpflegevorrichtung durch ihren einfachen Aufbau servicefreundlicher und die Herstellungskosten werden verringert.

Die Pendelwinkel einer jeden Fräswelle in vertikaler Richtung können beispielsweise ±10° oder mehr betragen.

Um die Fräswellen aktiv zum Durchfahren einer ebenen Piste in ihre kollineare Anordnung zurückzustellen, ist es von Vorteil, wenn zwischen den Fräsrahmen eine Rückstelleinrichtung angeordnet ist. Bei einer einfachen Ausführungsform ist die Rücksteileinrichtung zwischen den benachbarten Enden der Fräsrahmen angeordnet.

Bei einer Ausführungsform der Rückstelleinrichtung ist diese als elastischer Flachverbinder ausgebildet, welcher sich in Längsrichtung zwischen zwei Versteifungsrohren der Prallverkleidungen und in seiner Querrichtung im wesentlichen parallel zur Längsachse des Längslenkers erstreckt. Der Flachverbinder kann beispielsweise als Flachstahl oder aus einem Material mit ähnlichen Eigenschaften gebildet sein. Er erstreckt sich über die Lücke zwischen den beiden benachbarten Fräswellen von einem Versteifungsrohr bis in das andere.

Da der Flachverbinder mit seiner Querrichtung im wesentlichen parallel zur Längsachse des Längslenkers ausgerichtet ist, übt er insbesondere bei einem ungleichmäßigen Verschwenken um die horizontalen Längsachsen der entsprechenden Lagerhülsen Rückstellkräfte auf die Fräswellen aus.

Um die Fräswellenführung durch eine Pendel lagerung zu verbessern, ist es von Vorteil, wenn eine Drehachse der Fräswelle mit Abstand unterhalb des Längslenkers angeordnet ist.

Um den Tragrahmen am hinteren Ende zu versteifen, ist es günstig, wenn benachbart zu den freien Enden der hinteren U-Schenkel ein Versteifungsträger im wesentlichen parallel zum Querträger zwischen den U-Schenkeln angeordnet ist.

Zur lösbaren Befestigung und Lagerung des Glättbretts ist es insbesondere von Vorteil, wenn an den freien Enden der hinteren U-Schenkels querverlaufende Glättebrettleisten verschwenkbar gelagert sind. Diese erstrecken sich im wesentlichen parallel zu den Fräswellen und liegen auf einer Oberseite des Glättebretts auf. Die Lagerung kann in bekannter Weise sowohl um eine im wesentlichen horizontale Längs- und/oder um eine im wesentlichen horizontale Querachse erfolgen.

Die Glättebrettleisten können auch so ausgebildet sein, daß sie nur in der Einsatzstellung der Pistenpflegevorrichtung mit dem Glättebrett in Anlage sind und dieses auf den Pistenuntergrund aufdrücken. In diesem Fall ist es von Vorteil, wenn von den Glättebrettleisten wenigstens jeweils ein Glättebrettträger nach vorn absteht, dessen freies Ende am Glättebrett lösbar befestigt ist.

Bei einer Ausführungsform des Glättebretts ist dieses aus einem Glätteblatt, Abweiser und Tragkörper gebildet, wobei die Glättebrettträger am Tragkörper lösbar befestigt sind und von diesem der Abweiser im wesentlichen nach oben und das Glätteblatt im wesentlichen nach unten absteht. In diesem Fall liegt die Glättebrettleiste bei einer in Einsatzstellung angeordneten Pistenpflegevorrichtung auf der Oberfläche des Glätteblatts auf.

In diesem Zusammenhang ist es weiterhin günstig, wenn Glätteblatt und Abweiser lösbar am Tragkörper befestigt sind. Eine einfache Verbindung zwischen Tragkörper und Glätteblatt bzw. Abweiser kann beispielsweise über eine schwalbenschwanzähnliche Verbindung erfolgen. Abweiser und Tragkörper sind aus beispielsweise Kunststoff oder einem entsprechenden Metall herstellbar, wobei das Glätteblatt aus einem biegsamen Material hergestellt ist. Die Oberflächen von Glätteblatt, Abweiser und Tragkörper gehen bei einer vorteilhaften Ausführungsform fluchtend ineinander über.

Dabei ist es weiterhin munstig, wenn wenigstens der Abweiser entsprechend zur Schneefräsee mit zwei Fräswellen in Querrichtung aufgeteilt ist. Durch die Zweiteilung des Abweisers ist beispielsweise bei elastischer Ausführung des Tragkörpers eine Anpassung des Glättebretts an Unebenheiten der Piste entsprechend zur Anpassung der Fräswellen möglich.

Zur verbesserten Handhabung der Pistenpflegevorrichtung ist es weiterhin von Vorteil, wenn diese insgesamt symmetrisch zur in Längsrichtung verlaufenden Mittellinie ausgebildet ist.

Bei einer anderen Ausführungsform der Erfindung erstreckt sich der Querträger im wesentlichen zwischen den vorderen Enden der Längslenker und ist über in Richtung zur Kupplungseinrichtung abstehende Lagerlaschen an der horizontalen Querachse verschwenkbar gelagert. In diesem Fall sind die Längslenker beispielsweise L-förmig ausgebildet, wobei am freien Ende des längeren L-Schenkels der Querträger und die Schneefräse und am freien Ende des kürzeren L-Schenkels der Glättebrett angeordnet sind.

Um den Tragrahmen in der Einsatzstellung der Pistenpflegevorrichtung in Richtung der Pistenoberfläche zu drücken, ist es von Vorteil, wenn vom Querträger ein Anschlaghebel im wesentlichen nach oben absteht, der mit wenigstens einem seitlich an der Kupplungeinrichtung angeordneten Anlagearm in Anlage bringbar ist.

Ist die Schneefräse mit zwei Fräswellen ausgebildet, so ist es von Vorteil, wenn die Rückstelleinrichtung als seitlich zu einem Versteifungsrohr der Fräsrahmen verlaufender, mit diesen verbundener, elastischer Verbinder ausgebildet ist. Wie vorstehend schon erwähnt, verläuft der Flachverbinder in Querrichtung vertikal zur Schwenkebene der Fräswellen. Auf diese Weise wird der Flachverbinder bei einem Verschwenken der Fräswellen gegeneinander, d.h. bei einer unterschiedlichen Verdrehung der Längslenker um die horizontalen Längsachsen, aufgrund seiner Flexibilität gegen eine Rückstellkraft verbogen.

Bei einer weiteren Ausführungsform der Erfindung stehen vom Versteifungsquerträger nach hinten weisende Anschlagarme zur Anlage von vom Glättebrett abstehenden Anlagehebeln ab. Auf diese Weise wird ein Einstellwinkel des Glättebretts in Richtung zur Schneefräse festgelegt.

In diesem Zusammenhang ist es weiterhin von Vorteil, wenn zwischen dem freien Ende des Anschlagarms und dem Glättebrett eine Stelleinrichtung angeordnet ist. Mittels dieser Stelleinrichtung ist insbesondere das Glätteblatt des Glättebretts in Richtung zur Pistenoberfläche kraftbeaufschlagbar. Außerdem ist das Glättebrett von der Pistenoberfläche abhebbar.

Zur einfachen Befestigung der Stelleinrichtung an dem Glättebrett ist es günstig, wenn wenigstens eine Lagerlasche auf der Oberseite des Glättebretts ausgebildet ist.

Im folgenden werden vorteilhafte Ausführungsformen der Erfindung anhand der in der Zeichnung dargestellten Figuren näher erläutert und beschrieben. Es zeigen:
Fig. 1 eine Draufsicht auf eine Ausführungsform der erfindungsgemäßen Pistenpflegevorrichtung;
Fig. 2 einen Schnitt entlang der Linie II-II aus Fig. 1; und
Fig. 3 eine weitere Ausüfhrungsform der Erfindung in einer Darstellung entsprechend zur Fig. 2.

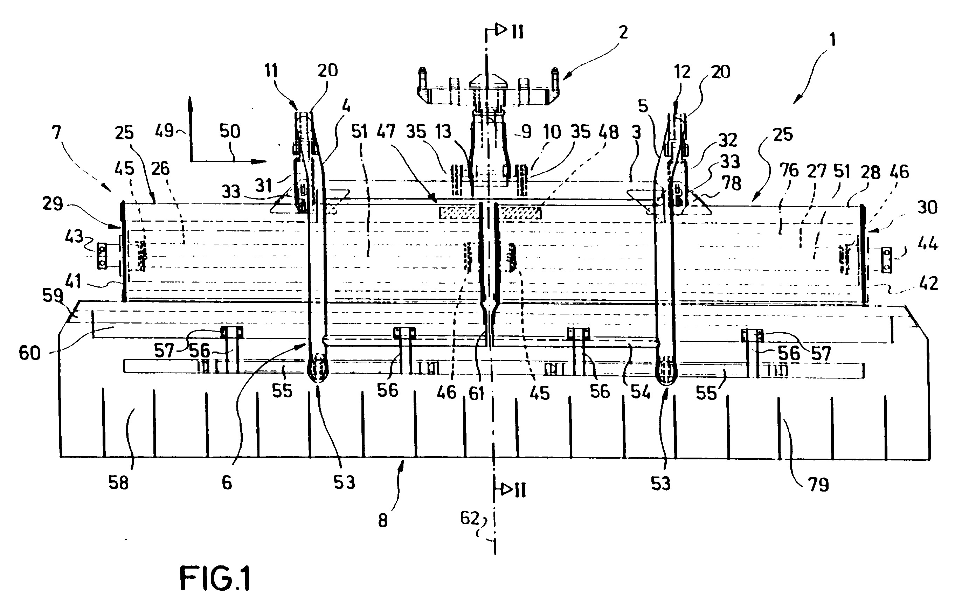

In Fig. 1 ist die Pistenpflegevorrichtung 1 in einer Draufsicht mit einer Kupplungseinrichtung 2, einer Schneefräse 7 und einem dieser nachgeordneten Glättebrett 8 dargestellt. Die Kupplungseinrichtung 2 dient zur Ankopplung der Pistenpflegevorrichtung 1 an ein nicht dargestelltes Fahrzeug. Sie ist an sich bekannt und wird im weiteren nur teilweise beschrieben. Die Kupplungseinrichtung ist über entsprechende Einrichtungen am Fahrzeug in bekannter Weise zum Verschwenken der Pistenpflegevorrichtung 1 aus einer Bereitschaftstellung in eine Einsatzstellung und umgekehrt an dem Fahrzeug verstellbar gelagert. Im hinteren Ende der Kupplungseinrichtung 2 ist eine im wesentlichen horizontale Längsachse 9 und eine im wesentlichen horizontale Querachse 10 ausgebildet, die zum Verschwenken von Schneefräse 7 und Glättebrett 8 dienen.

Zur Lagerung von Schneefräse 7 und Glättebrett 8 ist ein Tragrahmen 6 angeordnet, der sich im wesentlichen oberhalb von Schneefräse und Glättebrett erstreckt. Der Tragrahmen 6 umfaßt einen Querträger 3, zwei Längsträger 4 und 5 und einen Versteifungsträger 54. Querträger 3 und Versteifungsträger 54 sind parallel zueinander angeordnet und erstrecken sich in Querrichtung 50 der Pistenpflegevorrichtung 1. Der Querträger 3 ist an seiner Mitte 13 mittels zweier voneinander beabstandeter Lagerlaschen 35 an der Kupplungseinrichtung 2 um die im wesentlichen horizontale Querachse 10 verschwenkbar gelagert. Der Querträger 3 ist rohrförmige ausgebildet und endet auf den einander zuweisenden Seiten der Längsträger 4 und 5, wo er an diesen befestigt ist.

Die Längsträger 4 und 5 verlaufen im wesentlichen senkrecht zum Querträger 3 und Versteifungsträger 54, wobei sie sich in Längsrichtung 49 der Pistenpflegevorrichtung 1 sowohl in Richtung der Kupplungseinrichtung 2 als auch In Richtung zum Glättebrett 8 über Querträger 3 und Versteifungsträger 54 hinaus erstrecken. Die über den Querträger 3 nach vorne überstehenden Enden der Längsträger 4 und 5 sind unter einem spitzen Winkel gegenüber der Längsrichtung 49 nach außen, d.h. von der Kupplungseinrichtung 2 weg, abgeknickt. An ihren freien vorderen Enden 11 und 12 sind Lagerhülsen 20 angeordnet, an denen die Schneefräse 7 verschwenkbar gelagert ist. An ihren hinteren Enden 53 der Längsträger 4 und 5 sind zur Halterung des Glättebretts 8 Glättebrettleisten 55 verschwenkbar gelagert.

Die Schneefräse 7 ist zweiteilig ausgebildet und umfaßt eine linke Fräswelle 26 und eine rechte Fräswelle 27. Beide Fräswellen sind an ihren seitlichen Enden 45 und 46 in Fräsrahmen 25 drehbar gelagert. Die Fräswellen 26 und 27 sind mit ihren Drehachsen 51 parallel zum Querträger 3 oder Versteifungsträger 54 angeordnet. Von den Fräswellen stehen eine Vielzahl von Fräsmessern ab, die sich bis zur Umfangslinie 76 der Fräswellen 26 und 27 erstrecken. Zur Lagerung der Fräsrahmen am Tragrahmen 6 stehen von diesen unterhalb der Längsträger 4 und 5 und im wesentlichen parallel zu diesen Längslenker 31 und 32 in etwa mittig ab. Zur Verstärkuna der Verbindung von Längslenkern 31 und 32 und einer die Fräswellen 26 und 27 zumindest teilweise umgebenden Prallverkleidung 28 sind zwischen den beiden in etwa dreieckförmige Versteifungsbleche 78 angeordnet. Am der Prallverkleidung 28 gegenüberliegenden Ende sind die Längslenker 31 und 32 an den Lagerhülsen 20 an den freien Enden 11 und 12 der Längsträger 4 und 5 verschwenkbar gelagert.

Die Prallverkleidung 28 ist an ihren außenliegenden Enden 29 und 30 sowie an ihren zu einer Mittellinie 62 benachbarten Enden durch seitliche Schürzen verkleidet, die gleichzeitig zur Lagerung der Fräswellen 26 und 27 dienen. Zum getrennten Antrieb beider Fräswellen sind auf den außenliegenden Enden 29 und 30 der Prallverkleidung 28 Antriebseinrichtungen 43 und 44 für Fräswelle 26 bzw. 27 angeordnet. Diese Antriebseinrichtungen sind im wesentlichen konzentrisch zur Drehachse 51 der Fräswellen 26 und 27 angeordnet.

Benachbart zum Querträger 3 ist eine Rückstelleinrichtung 47 zwischen den beiden Prallverkleidungen 28 der Fräswellen 26 und 27 angeordnet. Die Rückstelleinrichtung ist aus einem Flachverbinder gebildet, der sich im wesentlichen parallel zum Querträger 3 erstreckt und teilweise beidseitig zur Mittellinie 62 in die Prallverkleidungen 28 eingesteckt ist.

Zwischen Längslenkern 31 und 32 und den Längsträgern 4 und 5 sind auf den dem Querträger 3 gegenüberliegenden Seitenflächen der Längsträger 4 und 5 Stelleinrichtungen 33 zur Höhenverstellung von Fräswelle 26 bzw. 27 angeordnet.

Die den hinteren Enden 53 der Längsträger 4 und 5 zugeordneten Glättebrettleisten 55 sind in etwa mittig um eine in Längsrichtung 49 verlaufende Achse an den Enden 53 verschwenkbar gelagert. Die Glättebrettleisten erstrecken sich entlang der Querrichtung 50 symmetrisch zu den Längsträgern 4 und 5. Benachbart zu deren Enden stehen Glättbrettträger 56 im wesentlichen rechtwinklig ab und sind an ihren freien Enden 57 am Glättebrett 8 lösbar befestigt.

Das Glättebrett 8 ist aus dem mit den Glättebrettträgern 57 lösbar verbundenen Tragkörper 60, einem Glätteblatt 58 und einem Abweiser 59 gebildet. Der Abweiser 59 verläuft benachbart und parallel zur Schneefräse 7, wobei er entlang der Mittellinie 62 durch eine Mittenteilung 61 unterbrochen ist. Das Glätteblatt 58 erstreckt sich vom Tragkörper 60 nach hinten, wobei die Glättebrettleisten 55 von oben auf dem Glätteblatt 58 lösbar befestigt sind. Die Breite des Glättebretts 8 ist größer als die Breite der Schneefräse 7, wobei durch die seitlichen Enden des Glättebretts 8 die seitlich an der Schneefräse 7 hervorstehenden Antriebseinrichtungen 43 und 44 überdeckt sind. Das Glätteblatt 58 weist eine Vielzahl von Teilungsschlitzen 79 auf, die parallel zueinander angeordnet sind und von dem hinteren Ende des Glätteblatts 58 in Richtung der Glättebrettleisten 55 verlaufen. Die Teilungsschlitze 79 teilen das Glätteblatt 58 in Querrichtung 50 in voneinander getrennte Abschnitte auf, die parallel zur Längsrichtung 49 gerichtet sind.

Die Pistenpflegevorrichtung 1 mit Schneefräse 7 und Glättebrett 8 ist symmetrisch zur Mittellinie 62 ausgebildet.

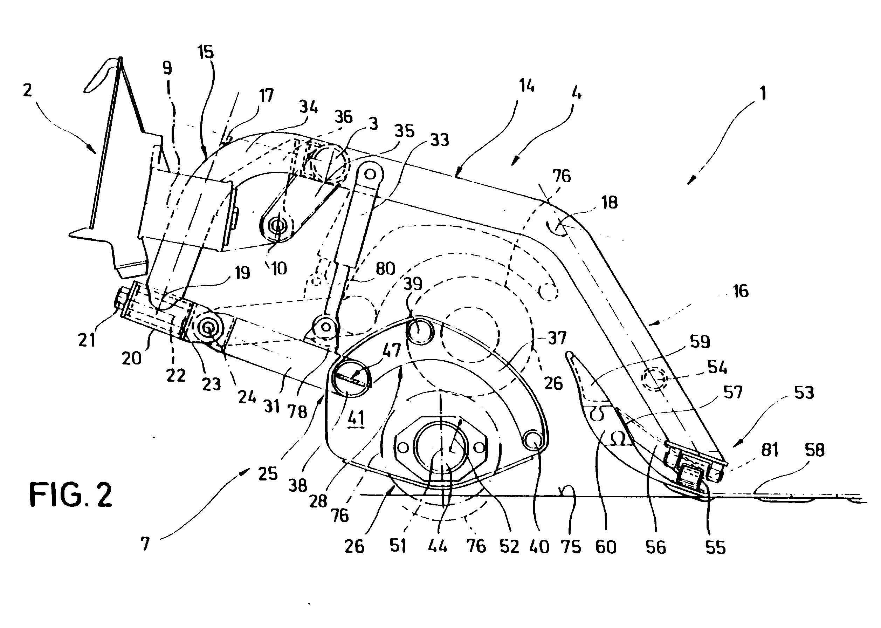

In Fig. 2 ist die Pistenpflegevorrichtung 1 entlang eines Schnitts II-II aus Fig. 1 dargestellt. Gleiche Bezugszeichen kennzeichnen gleiche Teile der Pistenpflegevorrichtung und werden nur nach teilweise erwähnt.

Der Längsträger 4 zeigt in Seitenansicht eine im wesentlichen U-Form. Ein U-Steg 14 verläuft oberhalb der Fräswelle 25 in Richtung der Kupplungseinrichtung 2 nach oben geneigt. An seinem der Kupplungseinrichtung 2 zugeordneten Ende ist der Querträger 3 angeordnet, der im wesentlichen die gleiche Höhe wie der U-Steg 14 aufweist. An den U-Steg 14 schließt sich in Richtung zur Kupplungseinrichtung 2 ein U-Schenkel 15 an, der in seinem geradlinigen Abschnnitt in etwa unter einem rechten Winkel 17 zum U-Steg 14 angeordnet ist. Zur Verbindung mit dem U-Steg 14 weist der U-Schenkel 15 einen in etwa 1/4-kreisförmig gekrümmten Verbindungsabschnitt 34 auf. Dieser geht benachbart zum Querträger 3 in den U-Steg 14 über. Am freien Ende 19 des U-Schenkels 15 ist an diesem die Lagerhülse 20 befestigt. Innerhalb der Lagerhülse ist ein Lagerbolzen 21 um die Längsachse 22 drehbar gelagert. Am hinteren Ende 23 des Lagerbolzens 21 ist mittels eines weiteren Lagerbolzens der Längslenker 31 um die im wesentlichen horizontale Querachse 24 verschwenkbar gelagert. Der Längslenker 31 ist mit seinem der Lagerhülse 20 zugeordneten Ende, siehe auch Fig. 1, zwischen zwei von der Lagerhülse abstehende Lagerlaschen gelagert.

Der Längslenker 31 ist an seinem der Lagerhülse 20 gegenüberliegenden Ende an der Prallverkleidung 28 befestigt. Diese ist aus einem in etwa halbkreisförmigen, die Fräswelle 25 nach oben abschirmenden Hohlprofil 37 gebildet. In diesem sind drei parallel zur Drehachse 51 der Fräswelle 25 verlaufende Versteifungsrohre 38, 39 ,und 40 angeordnet. Das Versteifungsrohr 38 mit dem größten Durchmesser ist im vorderen Ende der Prallverkleidung 37 eingesetzt und mit dem Längslenker 31 durch das zusätzliche Versteifungsbloch 78 verbunden. Innerhalb des Versteifungsronres 38 ist die Rückstelleinricntung 47 angeordnet. Nach Fig. 1 erstreckt sich die Rückstelleinrichtung in Längsrichtung im wesentlichen parallel zur Querrichtung 50 der Pistenpflegevorrichtung 1. Nach Fig. 2 erstreckt sich die Rückstelleinichtung 47 in ihrer Querrichtung parallel zur Längsrichtung des Längslenkers 31 und in etwa mittig zu diesem.

Direkt benachbart zur Prallverkleidung 28 ist auf dem Versteifungsblech 78 ein unteres Ende der Stelleinrichtung 33 verschwenkbar gelagert. Das gegenüberliegende obere Ende ist seitlich an dem U-Steg 14 verschwenkbar gelagert. Durch Variation des Ausschubs eines Kolbens 80 der Stelleinrichtung 33 erfolgt eine Höhenverstellung der Schneefräse 7. In Fig. 2 ist die Fräswelle 25 mit ihrer Umfangslinie 76 teilweise in einer Pistenoberfläche 75 versenkt dargestellt. Durch Betätigung der Stelleinrichtung 33 ist die Fräswelle 25 in die gestrichelt dargestellten Positionen höhenverstellbar.

Zur verbesserten Anpassung an den Verlauf des Längslenkers 4 verläuft die Prallverkleidung 37 zwischen dem Versteifungsrohr 39 und dem an ihrem hinteren Ende eingesetzten Versteifungsrohr 40 etwas gegenüber dem Abschnitt der Prallverkleidung zwischen den Versteifungsrohren 38 und 39 nach unten abgeknickt. Die der Fräswelle 25 zugeordnete Innenseite der Prallverkleidung 37 ist im wesentlichen halbkreisförmig ausgebildet. Die Drehachse 51 der Fräswelle 25 ist in einem Abstand 52 zum Längslenker 31 unterhalb einer durch dessen verlängerte, mittlere Längsachse gebildeten Achse angeordnet.

Die Prallverkleidung 28 ist an ihren seitlichen Enden durch eine Schürze 41 bzw. auf der anderen Seite durch eine Schürze 42 verkleidet. Die Schürzen dienen sowohl zur seitlichen Halterung der Versteifungsrohre 38, 39 und 40 als auch zur Lagerung der Fräswelle 25. Sie erstrecken sich entlang der Prallverkleidung 37 und gehen in diese am oberen Ende formschlüssig über. Nach unten überdecken die Schürzen die Fräswelle 25 zum größten Teil, wobei die Antriebseinrichtung 44 der Fräswelle 25 mittels zweier Schrauben an der Schürze befestigt ist. Das untere Ende der Schürze verläuft vom Versteifungsrohr 40 schräg nach unten bis unterhalb der Drehachse 51 und von dort in etwa parallel zum Längslenker 31 nach oben. Die dem Längslenker 31 zuweisende Vorderseite der Schürze verläuft in etwa senkrecht nach oben, wobei vom Versteifungsrohr 38 ab der Verlauf der Schürze dem der Prallverkleidung 37 entspricht.

Nach hinten schließt sich an den U-Steg 14 der U-Schenkel 16 an, wobei dieser unter einem stumpfen Winkel 18 nach hinten absteht. Der U-Schenkel 16 weist eine größere Länge als der U-Schenkel 15 auf und erstreckt sich bis nahe zur Pistenoberfläche 75. Benachbart zu seinem freien Ende 53 ist der Versteifungsquerträger 54 mit einer gegenüber dem Längsträger 4 geringeren Höhe angeordnet.

Am freien Ende 53 des U-Schenkels 16 ist die Glättebrettleiste 55 um eine zur Längsachse 22 der Lagerhülse 20 parallele Drehachse 81 verschwenkbar gelagert. Die Glättebrettleiste ist als Hohlprofil ausgebildet, das gegenüberliegend zum U-Schenkel 16 auf dem Glätteblatt 58 befestigt ist. In Richtung zur Kupplungseinrichtung 2 steht von der Glättebrettleiste 55 schräg nach oben der Glättebrettträger 56 ab. Dieser ist mit seinem freien Ende 57 am Tragkörper 60 lösbar befestigt. Der Tragkörper 60 weist in etwa einen parallelogrammförmigen Querschnitt auf, wobei die kürzeren Parallelogrammseiten schwalbenschwanzähnliche Nuten aufweisen, in denen entsprechende Vorsprünge von Glätteblatt 58 und Abweiser 59 eingreifen. Die längeren Parallelogrammseiten des Tragkörpers 60 sind auf der dem Ende 57 des Glättebrettträgers 56 zuweisenden Seite in etwa gerade und auf der gegenüberliegenden Seite in Richtung zur Fräswelle 25 konvex gewölbt. Diese Wölbung wird durch die Unterseite des Glätteblatts 58 in Richtung zur Pistenoberfläche 75 fortgesetzt, während die der Fräswelle 25 zuweisende Seite des Abweisers 59 konkav gekrümmt ist und formschlüssig in den Tragkörper 60 übergeht.

Unterhalb des Glätteblatts 58 sind eine Vielzahl von in Längsrichtung verlaufenden, bekannten Vorsprüngen angeordnet. Oberhalb des vordersten Vorsprungs ist die Glättebrettleiste 55 angeordnet, die den vorderen Abschnitt des Glätteblatts 58 unter einem spitzen Winkel relativ zur Pistenoberfläche 75 gestellt. Der der Glättebrettleiste 55 nachfolgende Abschnitt des Glättebretts 58 liegt auf der Pistenoberfläche 75 auf bzw. dringt mit den Vorsprüngen in die Pistenoberfläche ein.

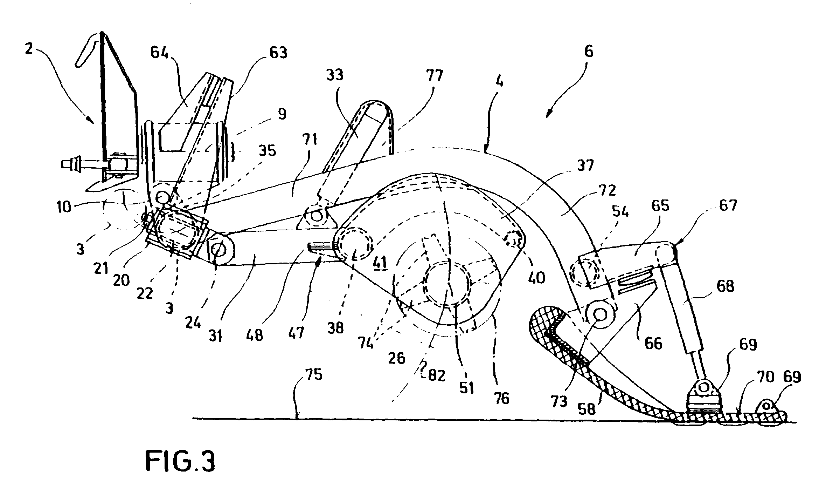

In Fig. 3 ist eine weitere Ausführungsform der erfindungsgemäßen. Pistenpflegevorrichtung dargestellt. Gleiche Bezugszeichen kennzeichnen wiederum gleiche Teile und werden nur noch teilweise dargestellt.

Der Tragrahmen 6 umfaßt Längsträger 4 und Querträger 3 sowie Versteifungsquerträger 54. Die Längsträger 4 sind im wesentlichen L-förmig ausgebildet, wobei am freien Ende des längeren L-Schenkels 71 der Querträger 3 die voneinander beabstandeten Längsträger 4 verbindet. Der kürzere L-Schenkel 72 ist über eine in etwa viertelkreisförmige Krümmung mit dem längeren L-Schenkel 71 verbunden, wobei an seinem freien Ende das Glätteblatt 58 verschwenkbar gelagert ist.

In Verlängerung des Querträgers 3 ist seitlich zum L-Schenkel 71 die Lagerhülse 20 zur drehbaren Lagerung des Lagerbolzens 21 um Längsachse 22 angeordnet. Analog zur Darstellung nach Fig. 2 ist am Ende des Lagerbolzens 21 der Längslenker 31 um die im wesentlichen horizontale Querachse 24 verschwenkbar gelagert.

In der in Fig. 3 dargestellten Stellung ist die Fräswelle 26 von der Pistenoberfläche 75 durch Stelleinrichtung 33 abgehoben. Die Stelleinrichtung 33 ist zwischen der Oberseite des Längslenkers 31 und einer vom L-Schenkel 71 nach oben abstehender Haltelasche 77 angeordnet. An ihren Enden ist die Stelleinrichtung 33 jeweils verschwenkbar gelagert.

Seitlich zum Längslenker 31 ist die von dem Hohlrohr 38 radial abstehende Rückstelleinrichtung 47 angeordnet. Der Flachverbinder 48 ist an seinen Enden von oben und unten durch vom Hohlrohr 38 abstehende Flansche gehalten. Die Länge der Flansche in Richtung des Längslenkers 31 entspricht der Breite des Flachverbinders 48.

Die Prallverkleidung 37 ist nach Fig. 3 durch zwei Versteifungsrohre 38 und 40 an ihren Enden versteift. Die Krümmung der Oberseite der Prallverkleidung 37 ist im Gegensatz zur Krümmung der Prallverkleidung 37 aus Fig. 2 gleichmäßig und in etwa viertelkreisförmig. Unterhalb der Prallverkleidung 37 ist in der seitlichen Schürze 41 die Fräswelle 26 mit einer Vielzahl von radial abstehenden Fräsmessern 74 drehbar gelagert. Durch die Stelleinrichtung 33 ist die Drehachse 51 der Fräswelle 26 entlang der Kreislinie 82 zur Höhenverstellung der Fräswelle bewegbar.

Die im wesentlichen horizontale Querachse 10 zur drehbaren Lagerung des Tragrahmens 6 ist oberhalb des Querträgers 3 angeordnet, wobei dieser über in Richtung der Kupplungseinrichtung 2 nach schräg vorne und oben abstehende Lagerlaschen 35 an der Querachse 10 gelagert ist. Von dem Querträger 3 steht in etwa senkrecht zur Längsachse 22 der Lagerhülse 20 ein Anschlaghebel 63 ab. Dieser weist im Querschnitt in etwa die Form eines rechtwinkligen Dreiecks auf. Am oberen Ende der längeren Kathete ist der Anschlaghebel 63 mit einem von der Kupplungseinrichtung 2 abstehenden Anlagearm 64 in Anlage. Dieser ist um einen spitzen Winkel gegenüber der Vertikalen nach hinten geneigt, wobei zwischen Anlagearm und Anschlaghebel ein Puffer angeordnet ist.

Am freien Ende des L-Schenkels 72 ist das Glätteblatt 58 um eine im wesentlichen horizontale Querachse 73 verschwenkbar gelagert. Das Glätteblatt 58 weist eine halbkreisförmige vordere Spitze auf, wobei zwischen dieser und dem sich nach hinten erstreckenden Glätteblatt 58 eine Stufe gebildet ist, in der ein Anlagehebel 66 angeordnet ist. Der Anlagehebel 66 ist mit seiner dem Glätteblatt 58 zugewandten Seite mit diesem lösbar verbunden und an der Querachse 73 in etwa mittig verschwenkbar gelagert. An seinem dem Glätteblatt 58 gegenüberliegenden Ende ist der Anlagehebel 66 mit einem vom Versteifungsquerträger 54 nach hinten oben abstehenden Anschlagarm 65 in Anlage. In etwa mittig zur Längsausdehnung des Anschlagarms 65 ist zur Anlage des freien Endes des Anlagehebels 66 ein Puffer angeordnet. An seinem freien Ende 67 ist am Anschlagarm 65 eine Stelleinrichtung 68 verschwenkbar gelagert. Diese ist mit ihrem unteren Ende an einer von der Oberseite 70 des Glätteblatts 58 abstehenden Lagerlasche 69 verbunden. Der Abschnitt des Glätteblatts 58 unterhalb der Stelleinrichtung 68 und das folgende Ende des Glätteblatts 58 liegt auf der Pistenoberfläche 75 auf. Der der Stelleinrichtung 68 vorgeordnete Abschnitt des Glätteblatts 58 ist durch Anlagehebel 66 und Anschlagarm 65 unter einem spitzen Anstellwinkel gegenüber der Pistenoberfläche 75 gehalten.

## Patentansprüche

1. Pistenpflegevorrichtung (1) zum Anbau an ein Fahrzeug mittels einer Kupplungseinrichtung (2) mit einem aus wenigstens einem Querträger (3) und zwei Längsträgern (4, 5) gebildeten Tragrahmen (6) an dem eine höhenverstellbare Schneefräse (7) und ein Glättebrett (8) gelagert sind, wobei der Tragrahmen (6) an der Kupplungseinrichtung (2) um eine im wesentlichen horizontale Längs- und eine im wesentlichen horizontale Querachse (9, 10) verschwenkbar gelagert ist, **dadurch gekennzeichnet,** daß die Schneefräse (7) geschleppt von vorderen Enden (11, 12) der Längsträger (4,5) an diesen verschwenkbar gelagert ist und der Querträger (3) zur Lagerung des Tragrahmens (6) der Kupplungseinrichtung (2) zugeordnet ist.

2. Pistenpflegevorrichtung nach Anspruch 1, **dadurch gekennzeichnet,** daß der Querträger (3) zwischen den Längsträgern (4, 5) verläuft und im wesentlichen mittig an der Kupplungseinrichtung (2) verschwenkbar gelagert ist.

3. Pistenpflegevorrichtung nach Anspruch 1 oder 2, **dadurch gekennzeichnet,** daß die Längsträger (4, 5) in einer vertikalen Ebene in etwa U-förmig mit jeweils einem U-Steg (14) und einem vorderen und einem hinteren U-Schenkel (15, 16) ausgebildet sind.

4. Pistenpflegevorrichtung nach wenigstens einem der vorangehenden Ansprüche, **dadurch gekennzeichnet,** daß der der Schneefläse (7) zugeordnete, vordere U-Schenkel (15) im wesentlichen einen rechten Winkel (17) und der dem Glättebrett (8) zugeordnete, hintere U-Schenkel (16) einen stumpfen Winkel (18) mit dem U-Steg (14) einschließt.

5. Pistenpflegevorrichtung nach wenigstens einem der vorangehenden Ansprüche, **dadurch gekennzeichnet,** daß am freien Ende (19)) des vorderen U-Schenkels (15) zur verschwenkbaren Lagerung der Schneefräse (7) eine Lagerhülse (20) zur Aufnahme eines um einen im wesentlichen horizontale Längsachse (22) verschwenkbaren Lagerbolzen (21) angeordnet ist, an dessen hinterem Ende (23) die Schneefräse (7) um eine im wesentlichen horizontale Querachse (24) verschwenkbar gelagert ist.

6. Pistenpflegevorrichtung nach wenigstens einem der vorangehenden Ansprüche, **dadurch gekennzeichnet,** daß die Schneefräse (7) einen Fräsrahmen (25) und wenigstens eine am Fräsrahmen drehbar gelagerte Fräswelle (26, 27) umfaßt, wobei der Fräsrahmen (25) an den vorderen U-Schenkeln (15) angelenkt ist.

7. Pistenpflegevorrichtung nach wenigstens einem der vorangehenden Ansprüche, **dadurch gekennzeichnet,** daß der Fräsrahmen (25) eine im wesentlichen halbkreisförmige Prallverkleidung (28), an deren seitlichen Enden (29, 30) die Fräswelle (26, 27) drehbar gelagert ist, und wenigstens zwei zwischen den U-Schenkelenden (19) und der Prallverkleidung (28) angeordnete Längslenker (31, 32) aufweist.

8. Pistenpflegevorrichtung nach wenigstens einem der vorangehenden Ansprüche, **dadurch gekennzeichnet,** daß zwischen Längslenker (31, 32) und U-Steg (14) wenigstens eine Stelleinrichtung (33) zur Höhenverstellung der Schneefräse (7) angeordnet ist.

9. Pistenpflegevorrichtung nach wenigstens einem der vorangehenden Ansprüche, **dadurch gekennzeichnet,** daß der vordere U-Schenkel (15) mit dem U-Steg (14) über einen unter einem spitzen Winkel nach außen abgeknickten Verbindungsabschnitt (34) verbunden ist.

10. Pistenpflegevorrichtung nach wenigstens einem der vorangehenden Ansprüche, **dadurch gekennzeichnet,** daß der Querträger (3) benachbart zu den Verbindungsabschnitten (34) der vorderen U-Schenkel (15) zwischen den U-Stegen (14) angeordnet ist und an der Kupplungseinrichtung (2) um die im wesentlichen horizontale Querachse (10) verschwenkbar gelagert ist.

11. Pistenpflegevorrichtung nach wenigstens einem der vorangehenden Ansprüche, **dadurch gekennzeichnet,** daß der Querträger (3) über zwei in etwa in Richtung des freien Endes (19) der vorderen U-Schenkel (15) verlaufende Lagerlaschen (35) an der Kupplungseinrichtung (2) gelagert ist.

12. Pistenpflegevorrichtung nach wenigstens einem der vorangehenden Ansprüche, **dadurch gekennzeichnet,** daß an der Kupplungseinrichtung (2) ein Anschlag (36) für den Querträger (3) angeordnet ist.

13. Pistenpflegevorrichtung nach wenigstens einem der vorangehenden Ansprüche, **dadurch gekennzeichnet,** daß die Prallverkleidung (28) der Fräswelle (26, 27) aus einem Hohlprofil (37) mit in diesen im wesentlichen prallel zur Fräswelle verlaufenden Versteifungsrohren (38, 39, 40) gebildet ist, wobei die Prallverkleidung (28) seitlich durch im wesentlichen senkrecht zur Fräswelle verlaufende Schürzen (41, 42) abgedeckt ist.

14. Pistenpflegevorrichtung nach wenigstens einem der vorangehenden Ansprüche, **dadurch gekennzeichnet,** daß die Schneefräse (7) aus zwei Fräswellen (26, 27) mit entsprechenden Fräsrahmen (25) gebildet ist, wobei die Längslenker (31, 32) eines jeden Fräsrahmens (25) in Querrichtung (50) im wesentlichen mittig an diesen angeordnet sind.

15. Pistenpflegevorrichtung nach wenigstens einem der vorangehenden Ansprüche, **dadurch gekennzeichnet,** daß jeder Fräswelle (26, 27) eine Antriebseinrichtung (43, 44) zugeordnet ist.

16. Pistenpflegevorrichtung nach wenigstens einem der vorangehenden Ansprüche, **dadurch gekennzeichnet,** daß die Antriebseinrichtungen (43, 44) an außenliegenden, seitlichen Enden (29, 30) der Fräswellen (26, 27) angeordnet sind.

17. Pistenpflegevorrichtung nach wenigstens einem der vorangehenden Ansprüche, **dadurch gekennzeichnet,** daß zwischen den Fräsrahmen (25) eine Rückstelleinrichtung (47) angeordnet ist.

18. Pistenpflegevorrichtung nach wenigstens einem der vorangehenden Ansprüche, **dadurch gekennzeichnet,** daß die Rückstelleinrichtung (47) zwischen den benachbarten Enden (46) der Fräsrahmen (25) angeordnet ist.

19. Pistenpflegevorrichtung nach wenigstens einem der vorangehenden Ansprüche, **dadurch gekennzeichnet,** daß die Rückstelleinrichtung (47) als elastischer Flachverbinder ausgebildet ist, welcher sich in Längsrichtung zwischen zwei Versteifungsrohren (38) der Prallverkleidungen (28) und in seiner Querrichtung im wesentlichen parallel zur horizontalen Längsachse (22) der Lagerhülse (20) erstreckt.

20. Pistenpflegevorrichtung nach wenigstens einem der vorangehenden Ansprüche, **dadurch gekennzeichnet,** daß eine Drehachse (51) der Fräswelle (26, 27) mit Abstand (52) unterhalb des Längslenkers (4, 5) angeordnet ist.

21. Pistenpflegevorrichtung nach wenigstens einem der vorangehenden Ansprüche, **dadurch gekennzeichnet,** daß benachbart zu den freien Enden (53) der hinteren U-Schenkel (16) ein Versteifungsträger (54) im wesentlichen parallel zum Querträger (3) angeordnet ist.

22. Pistenpflegevorrichtung nach wenigstens einem der vorangehenden Ansprüche, **dadurch gekennzeichnet,** daß am freien Ende (53) des hinteren U-Schenkels (16) querverlaufende Glättebrettleisten (55) verschwenkbar gelagert sind.

23. Pistenpflegevorrichtung nach wenigstens einem der vorangehenden Ansprüche, **dadurch gekennzeichnet,** daß von den Glättebrettleisten (55) wenigstens jeweils ein Glättebrettträger (56) nach vorn absteht, dessen freies Ende (57) am Glättebrett (8) lösbar befestigt ist.

24. Pistenpflegevorrichtung nach wenigstens einem der vorangehenden Ansprüche, **dadurch gekennzeichnet,** daß das Glättebrett (8) aus Glätteblatt (58), Abweiser (59) und Tragkörper (60) gebildet ist, wobei die Glättebrettträger (56) am Tragkörper (60) lösbar befestigt sind und von diesem der Abweiser (59) im wesentlichen nach oben und das Glätteblatt (58) im wesentlichen nach unten absteht.

25. Pistenpflegevorrichtung nach wenigstens einem der vorangehenden Ansprüche, **dadurch gekennzeichnet,** daß das Glätteblatt (58) und Abweiser (59) lösbar am Tragkörper (60) befestigt sind.

26. Pistenpflegevorrichtung nach wenigstens einem der vorangehenden Ansprüche, **dadurch gekennzeichnet,** daß wenigstens der Abweiser (59) entsprechend zur Schneefräsee (7) mit zwei Fräswellen (26, 27) in Querrichtung (50) aufgeteilt ist.

27. Pistenpflegevorrichtung nach wenigstens einem der vorangehenden Ansprüche, **dadurch gekennzeichnet,** daß die Pistenpflegevorrichtung (1) symmetrisch zur in Längsrichtung (49) verlaufenden Mittellinie (62) ausgebildet ist.

28. Pistenpflegevorrichtung nach wenigstens einem der vorangehenden Ansprüche, **dadurch gekennzeichnet,** daß sich der Querträger (30) im wesentlichen zwischen den vorderen Enden (11, 12) der Längsträger (4, 5) erstreckt und über in Richtung zur Kupplungseinrichtung (2) abstehende Lagerlaschen (35) an der horizontalen Querachse (10) verschwenkbar gelagert ist.

29. Pistenpflegevorrichtung nach wenigstens einem der vorangehenden Ansprüche, **dadurch gekennzeichnet,** daß vom Querträger (3) ein Anschlaghebel (63) im wesentlichen nach oben absteht, der mit einem seitlich an der Kupplungeinrichtung (2) angeordneten Anlagearm (64) in Anlage bringbar ist.

30. Pistenpflegevorrichtung nach wenigstens einem der vorangehenden Ansprüche, **dadurch gekennzeichnet,** daß die Rückstelleinrichtung (47) als seitlich zu einem Versteifungsrohr (38) der Fräsrahmen (25) verlaufender, mit diesen verbundener, elastischer Flachverbinder (48) ausgebildet ist.

31. Pistenpflegevorrichtung nach wenigstens einem der vorangehenden Ansprüche, **dadurch gekennzeichnet,** daß vom Versteifungsquerträger (54) ein nach hinten weisender Anschlagarm (65) zur Anlage eines vom Glättebrett (8) abstehenden Anlagehebels (66) absteht.

32. Pistenpflegevorrichtung nach wenigstens einem der vorangehenden Ansprüche, **dadurch gekennzeichnet,** daß zwischen dem freien Ende (67) des Anschlagarms (65) und dem Glättebrett (8) eine Stelleinrichtung (68) angeordnet ist.

33. Pistenpflegevorrichtung nach wenigstens einem der vorangehenden Ansprüche, **dadurch gekennzeichnet,** daß zur lösbaren Befestigung der Stelleinrichtung (68) wenigstens eine Lagerlasche (69) auf der Oberseite (70) des Glättebretts (8) ausgebildet ist.

## Claims

1. Ski slope maintenance device (1) for attachment to a vehicle by means of a coupling arrangement (2), with a support frame (6), formed by at least one crossbeam (3) and two longitudinal beams (4,5), on which a vertically adjustable rotary snowplough (7) and a smoothing blade assembly (8) are mounted, the support frame (6) being mounted on the coupling arrangement (2) so as to be tiltable about an essentially horizontal longitudinal axis (9) and an essentially horizontal transverse axis (10), characterized in that the rotary snowplough (7) is pivotably mounted on forward ends (11,12) of the longitudinal beams (4,5) so that it is pulled thereby and the crossbeam (3) is utilized for mounting the support frame (6) on the coupling arrangement (2).

2. Ski slope maintenance device according to claim 1, characterized in that the crossbeam (3) extends between the longitudinal beams (4,5) and has an essentially central pivotable mounting on the coupling arrangement (2).

3. Ski slope maintenance device according to claim 1 or claim 2, characterized in that the longitudinal beams (4,5) are approximately in the form of a U in a vertical plane, each having a U-web (14) and forward and rear U-legs (15,16).

4. Ski slope maintenance device according to at least one of the preceding claims, characterized in that the forward U-leg (15), which carries the rotary snowplough (7), includes an essentially 90° angle (17) with the U-web (14), and the rear U-leg (16), which carries the smoothing blade assembly (8), includes an obtuse angle (18) with the U-web (14).

5. Ski slope maintenance device according to at least one of the preceding claims, characterized in that the rotary snowplough (7) is given a pivotable mounting by arranging a bearing sleeve (20) on the free end (19) of the forward U-leg (15) to receive a bearing pin (21) which is pivotable about an essentially horizontal longitudinal axis (22) and to the rear end (23) of which the rotary snowplough (7) is hinged about an essentially horizontal transverse axis (24).

6. Ski slope maintenance device according to at least one of the preceding claims, characterized in that the rotary snowplough (7) comprises a cutter frame (25) and at least one cutter shaft (26,27) rotatably mounted on the cutter frame, the cutter frame (25) being linked to the forward U-legs (15).

7. Ski slope maintenance device according to at least one of the preceding claims characterized in that the cutter frame (25) has an essentially semicircular deflector guard (28) with the cutter shaft (26,27) rotatably mounted at the lateral ends (29,30) thereof, and at least two longitudinal link-rods (31,32) arranged between the ends of the U-legs (19) and the deflector guard (28).

8. Ski slope maintenance device according to at least one of the preceding claims, characterized in that at least one actuator (33) for vertical positioning of the rotary snowplough (7) is arranged between longitudinal link-rod (31,32) and U-web (14).

9. Ski slope maintenance device according to at least one of the preceding claims characterized in that the forward U-leg (15) is joined to the U-web (14) by a connecting portion (34) which is cranked outwards at an acute angle.

10. Ski slope maintenance device according to at least one of the preceding claims, characterized in that the crossbeam (3) is located between the U-webs (14) and close to the connecting portions (34) to the forward U-legs (15) and is mounted on the coupling arrangement (2) so that it is pivotable about the essentially horizontal transverse axis (10).

11. Ski slope maintenance device according to at least one of the preceding claims, characterized in that the crossbeam (3) is mounted on the coupling arrangement (2) by two bearing shackles (35) extending approximately towards the free end (19) of the forward U-legs (15).

12. Ski slope maintenance device according to at least one of the preceding claims, characterized in that a stop (36) for the crossbeam (3) is provided on the coupling arrangement (2).

13. Ski slope maintenance device according to at least one of the preceding claims, characterized in that the deflector guard (28) for the cutter shaft (26,27) is formed from a hollow section (37) with stiffening tubes (38,39,40) extending essentially parallel with the cutter shaft contained therein, the deflector guard (28) being closed off laterally by skirts (41,42) extending essentially perpendicularly to the cutter shaft.

14. Ski slope maintenance device according to at least one of the preceding claims, characterized in that the rotary snowplough (7) is formed from two cutter shafts (26,27) with their respective cutter frames (25), the longitudinal link-rod (31,32) of each cutter frame (25) being located essentially centrally on the frame, in the transverse direction (50).

15. Ski slope maintenance device according to at least one of the preceding claims, characterized in that each cutter shaft (26,27) is provided with a drive unit (43,44).

16. Ski slope maintenance device according to at least one of the preceding claims, characterized in that the drive units (43,44) are mounted at outlying lateral ends (29,30) of the cutter shafts (26,27).

17. Ski slope maintenance device according to at least one of the preceding claims, characterized in that a resetting device (47) is provided between the cutter frames (25).

18. Ski slope maintenance device according to at least one of the preceding claims, characterized in that the resetting device (47) is located between the adjacent ends (46) of the cutter frames (25).

19. Ski slope maintenance device according to at least one of the preceding claims, characterized in that the resetting device (47) is in the form of a flat elastic connector which in the longitudinal direction extends between two stiffening tubes (38) of the deflector guards (28) and in its transverse direction extends essentially parallel with the horizontal longitudinal axis (22) of the bearing sleeve (20).

20. Ski slope maintenance device according to at least one of the preceding claims, characterized in that an axis of rotation (51) of the cutter shaft (26,27) is located some distance (52) below the longitudinal link-rod (4,5).

21. Ski slope maintenance device according to at least one of the preceding claims, characterized in that a stiffening girder (54) essentially parallel with the crossbeam (3) is located adjacent to the free ends (53) of the rear U-legs (16).

22. Ski slope maintenance device according to at least one of the preceding claims, characterized in that transverse smoothing blade bars (55) are pivotably mounted at the free end (53) of the rear U-leg (16).

23. Ski slope maintenance device according to at least one of the preceding claims, characterized in that at least one smoothing blade bracket (56), with its free end (57) releasably attached to the smoothing blade assembly (8), projects forwards from each of the smoothing blade bars (55).

24. Ski slope maintenance device according to at least one of the preceding claims, characterized in that the smoothing blade assembly (8) is made up of a smoothing blade (58), deflector (59) and carrier (60), the smoothing blade brackets (56) being releasably attached to the carrier (60) and the deflector (59) projecting essentially upwards from the carrier and the smoothing blade (58) projecting essentially downwards from the carrier.

25. Ski slope maintenance device according to at least one of the preceding claims, characterized in that the smoothing blade (58) and deflector (59) are releasably attached to the carrier (60).

26. Ski slope maintenance device according to at least one of the preceding claims, characterized in that at least the deflector (59) is divided in the transverse direction (50), in line with the rotary snowplough (7) with two cutter shafts (26,27).

27. Ski slope maintenance device according to at least one of the preceding claims, characterized in that the ski slope maintenance device (1) is made symmetrical with respect to the centreline (62) extending in the longitudinal direction (49).

28. Ski slope maintenance device according to at least one of the preceding claims, characterized in that the crossbeam (30) extends essentially between the forward ends (11,12) of the longitudinal beams (4,5) and is mounted so as to be pivotable on the horizontal transverse axis (10) by bearing shackles (35) projecting towards the coupling arrangement (2).

29. Ski slope maintenance device according to at least one of the preceding claims, characterized in that a stop arm (63) projects essentially upwards from the crossbeam (3) and is able to be contacted by a contact arm (64) laterally located on the coupling arrangement (2).

30. Ski slope maintenance device according to at least one of the preceding claims, characterized in that the resetting device (47) is constructed as a flat elastic connector (48) extending laterally with respect to a stiffening tube (38) of the cutter frames (25), and connected thereto.

31. Ski slope maintenance device according to at least one of the preceding claims, characterized in that a rearwards pointing stop arm (65) projects from the stiffening cross girder (54) for abutment against a contact arm (66) projecting from the smoothing blade assembly (8).

32. Ski slope maintenance device according to at least one of the preceding claims, characterized in that an actuator (68) is located between the free end (67) of the stop arm (65) and the smoothing blade assembly (8).

33. Ski slope maintenance device according to at least one of the preceding claims, characterized in that for releasable attachment of the actuator (68) at least one bearing bracket (69) is formed on the upper side (70) of the smoothing blade assembly (8).

## Revendications

1. Dispositif (1) pour entretenir des pistes de ski à monter sur un véhicule au moyen d'un mécanisme d'accouplement (2) comprenant un cadre de support (6) formé par au moins un support transversal (3) et par deux supports longitudinaux (4, 5), auquel sont montés un chasse-neige à fraise (7) réglable en hauteur et une planche à lisser (8), le cadre de support (6) étant monté sur le mécanisme d'accouplement (2) de manière à pouvoir pivoter autour d'un axe longitudinal essentiellement horizontal et d'un axe transversal essentiellement horizontal (9, 10), caractérisé en ce que le chasse-neige à fraise (7) est monté en pivotement aux extrémités avant (11, 12) des supports longitudinaux (4, 5) pour être traîné par ces dernières, et le support transversal (3) est attribué au mécanisme d'accouplement (2) pour le montage du cadre de support (6).

2. Dispositif pour entretenir des pistes de ski selon le revendication 1, caractérisé en ce que le support transversal (3) s'étend entre les supports longitudinaux (4, 5) et est monté en pivotement essentiellement au milieu du mécanisme d'accouplement (2).

3. Dispositif pour entretenir des pistes de ski selon la revendication 1 ou 2, caractérisé en ce que les supports longitudinaux (4, 5) sont réalisés dans un plan vertical sous une forme correspondant approximativement à celle d'un U avec respectivement une barre (14) du U et une branche antérieure et une branche postérieure (15, 16) du U.

4. Dispositif pour entretenir des pistes de ski selon au moins une des revendications ci-dessus, caractérisé en ce que la branche antérieure (15) du U attribuée au chasse-neige à fraise (7) forme essentiellement un angle droit (17) avec la barre (14) du U et la branche postérieure (16) du U attribuée à la planche à lisser (8) forme un angle obtus (18) avec la barre (14) du U.

5. Dispositif pour entretenir des pistes de ski selon au moins une des revendications précédentes, caractérisé en ce que, à l'extrémité libre (19) de la branche antérieure (15) du U, est disposé, pour le montage en pivotement du chasse-neige à fraise (7), un coussinet (20) dans lequel vient se loger un boulon de palier (21) de manière à pouvoir pivoter autour d'un axe (22) essentiellement horizontal, à l'extrémité arrière (23) duquel est monté le chasse-neige à fraise (7) de manière à pouvoir pivoter autour d'un axe transversal (24) essentiellement horizontal.

6. Dispositif pour entretenir des pistes de ski selon au moins une des revendications précédentes, caractérisé en ce que le chasse-neige à fraise (7) comprend un cadre de fraise (25) et au moins une broche porte-fraise (26, 27) montée en rotation sur le cadre de fraise, dans lequel le cadre de fraise (25) est monté en articulation sur les branches antérieures (15) des U.

7. Dispositif pour entretenir des pistes de ski selon au moins une des revendications précédentes, caractérisé en ce que le cadre de fraise (25) présente une garniture de rebondissement (28) de forme essentiellement semi-circulaire, aux extrémités latérales (29, 30) de laquelle est montée en rotation la broche porte-fraise (26, 27), et au moins deux bielles longitudinales (31, 32) disposées entre les extrémités (19) des branches du U et la garniture de rebondissement (28).

8. Dispositif pour entretenir des pistes de ski selon au moins une des revendications précédentes, caractérisé en ce que, entre les bielles longitudinales (31, 32) et la barre (14) du U, est disposé au moins un mécanisme de réglage (33) pour le réglage en hauteur du chasse-neige à fraise (7).

9. Dispositif pour entretenir des pistes de ski selon au moins une des revendications précédentes, caractérisé en ce que la branche antérieure (15) du U est reliée à la barre (14) du U via une section de liaison (34) pliée vers l'extérieur en formant un angle aigu.

10. Dispositif pour entretenir des pistes de ski selon au moins une des revendications précédentes, caractérisé en ce que le support transversal (3) est disposé entre les barres (14) des U à proximité des sections de liaison (34) des branches antérieures (15) des U et est monté contre le mécanisme d'accouplement (2) de manière à pouvoir pivoter autour de l'axe transversal (10) essentiellement horizontal.

11. Dispositif pour entretenir des pistes de ski selon au moins une des revendications précédentes, caractérisé en ce que le support transversal (3) est monté sur le mécanisme d'accouplement (2) via deux attaches (35) faisant office de palier s'étendant approximativement dans la direction de l'extrémité libre (19) des branches antérieures (15) des U.

12. Dispositif pour entretenir des pistes de ski selon au moins une des revendications précédentes, caractérisé en ce qu'une butée (36) pour le support transversal (3) est disposée sur le mécanisme d'accouplement (2).

13. Dispositif pour entretenir des pistes de ski selon au moins une des revendications précédentes, caractérisé en ce que la garniture de rebondissement (28) de la broche porte-fraise (26, 27) est formée à partir d'un profilé creux (37) comprenant des tubes de renforcement (38, 39, 40) s'étendant dans ce dernier essentiellement parallèlement à la broche porte-fraise, la garniture de rebondissement (28) étant recouverte latéralement par des jupes (41, 42) s'étendant essentiellement perpendiculairement à la broche porte-fraise.

14. Dispositif pour entretenir des pistes de ski selon au moins une des revendications précédentes, caractérisé en ce que le chasse-neige à fraise (7) est formé à partir de deux broches porte-fraises (26, 27) munies de cadres de fraises correspondants (25), dans lequel les bielles longitudinales (31, 32) de chaque cadre de fraise (25) sont disposées en direction transversale (50) essentiellement au milieu de ce dernier.

15. Dispositif pour entretenir des pistes de ski selon au moins une des revendications précédentes, caractérisé en ce qu'un mécanisme de commande (43, 44) est attribué à chaque broche porte-fraise (26, 27).

16. Dispositif pour entretenir des pistes de ski selon au moins une des revendications précédentes, caractérisé en ce que les mécanismes de commande (43, 44) sont disposés aux extrémités latérales externes (29, 30) des broches porte-fraises (26, 27).

17. Dispositif pour entretenir des pistes de ski selon au moins une des revendications précédentes, caractérisé en ce qu'un mécanisme de rappel (47) est disposé entre les cadres de fraises (25).

18. Dispositif pour entretenir des pistes de ski selon au moins une des revendications précédentes, caractérisé en ce que le mécanisme de rappel (47) est disposé entre les extrémités voisines (46) des cadres de fraises (25).

19. Dispositif pour entretenir des pistes de ski selon au moins une des revendications précédentes, caractérisé en ce que le mécanisme de rappel (47) est réalisé sous forme d'un raccord horizontal élastique qui s'étend, en direction longitudinale, entre deux tubes de renforcement (38) des garnitures de rebondissement (28) et, dans sa direction transversale, essentiellement parallèlement à l'axe longitudinal horizontal (22) du coussinet (20).

20. Dispositif pour entretenir des pistes de ski selon au moins une des revendications précédentes, caractérisé en ce qu'un axe de rotation (51) des broches porte-fraises (26, 27) est disposé à une distance (52) en dessous de la bielle longitudinale (4, 5).

21. Dispositif pour entretenir des pistes de ski selon au moins une des revendications précédentes, caractérisé en ce que, à proximité des extrémités libres (53) des branches postérieures (16) des U, est disposé un support de renforcement (54) en position essentiellement parallèle au support transversal (3).

22. Dispositif pour entretenir des pistes de ski selon au moins une des revendications précédentes, caractérisé en ce que, à l'extrémité libre (53) de la branche postérieure (16) du U, sont montées en pivotement des lattes (55) pour la planche à lisser, s'étendant en direction transversale.

23. Dispositif pour entretenir des pistes de ski selon au moins une des revendications précédentes, caractérisé en ce que en ce qu'au moins respectivement un support (56) pour la planche à lisser fait saillie vers l'avant par rapport aux lattes (55) pour la planche à lisser, dont l'extrémité libre (57) est fixée de manière amovible à la planche à lisser (8).

24. Dispositif pour entretenir des pistes de ski selon au moins une des revendications précédentes, caractérisé en ce que la planche à lisser (8) est formée à partir d'une lame à lisser (58), d'un déflecteur (59) et d'un corps de support (60), les supports (56) pour la planche à lisser sont fixés de manière amovible au corps de support (60) et le déflecteur (59) fait saillie par rapport à ce dernier essentiellement vers le haut, la lame à lisser (58) faisant saillie essentiellement vers le bas.

25. Dispositif pour entretenir des pistes de ski selon au moins une des revendications précédentes, caractérisé en ce que la lame à lisser (58) et le déflecteur (59) sont fixés de manière amovible au corps de support (60).

26. Dispositif pour entretenir des pistes de ski selon au moins une des revendications précédentes, caractérisé en ce qu'au moins le déflecteur (59) est subdivisé en direction transversale (50) de manière correspondante au chasse-neige à fraise (7) muni de deux broches porte-fraises (26, 27).

27. Dispositif pour entretenir des pistes de ski selon au moins une des revendications précédentes, caractérisé en ce que le dispositif (1) pour entretenir des pistes de ski est réalisé symétriquement par rapport à la ligne médiane (62) s'étendant en direction longitudinale (49).

28. Dispositif pour entretenir des pistes de ski selon au moins une des revendications précédentes, caractérisé en ce que le support transversal (30) s'étend essentiellement entre les extrémités avant (11, 12) des supports longitudinaux (4, 5) et est monté en pivotement sur l'axe transversal horizontal (10) via des attaches (35) faisant office de palier faisant saillie par rapport au mécanisme d'accouplement (2).

29. Dispositif pour entretenir des pistes de ski selon au moins une des revendications précédentes, caractérisé en ce qu'un levier d'arrêt (63) fait saillie essentiellement vers le haut par rapport au support transversal (3), qui peut venir se mettre en contact avec le bras d'arrêt (64) disposé latéralement sur le mécanisme d'accouplement (2).

30. Dispositif pour entretenir des pistes de ski selon au moins une des revendications précédentes, caractérisé en ce que le mécanisme de rappel (47) est réalisé sous forme d'un raccord horizontal élastique (48) s'étendant latéralement par rapport à un tube de renforcement (38) du cadre de fraise (25) en étant relié à ce dernier.

31. Dispositif pour entretenir des pistes de ski selon au moins une des revendications précédentes, caractérisé en ce qu'un bras d'arrêt (65) orienté vers l'arrière pour la mise en contact avec un levier d'arrêt (66) faisant saillie par rapport à la planche à lisser (8), fait saillie par rapport au support transversal de renforcement (54).

32. Dispositif pour entretenir des pistes de ski selon au moins une des revendications précédentes, caractérisé en ce qu'un mécanisme de réglage (68) est disposé entre l'extrémité libre (67) du bras d'arrêt (65) et la planche à lisser (8).

33. Dispositif pour entretenir des pistes de ski selon au moins une des revendications précédentes, caractérisé en ce que, pour la fixation amovible du mécanisme de réglage (68), au moins une attache (69) faisant office de palier est réalisée sur le côté supérieur (70) de la planche à lisser (8).
